# EUROPEAN PATENT APPLICATION

(11) **EP 4 659 728 A1**
(43) Date of publication of application: **10.12.2025**
(21) Application number: 24749733.2
(22) Date of filing: 01.02.2024
(51) Int. Cl.: A61J 1/10, A61M 5/14

(54) **MULTI-CHAMBER CONTAINER FOR EYE DROPS, AND MANUFACTURING METHOD THEREFOR**

(30) Priority: 03.02.2023 CN 202310080935
(71) Applicant: Ocumension Therapeutics (Suzhou) Co., Ltd., Suzhou, Jiangsu 215124 (CN)
(72) Inventor: LIU, Ye, Suzhou, Jiangsu 215124 (CN); ZHANG, Zaijuan, Suzhou, Jiangsu 215124 (CN); CUI, Siqi, Suzhou, Jiangsu 215124 (CN)
(74) Representative: AWA Denmark A/S
(86) International application number: PCT/CN2024/075262
(87) International publication number: WO 2024/160254

(57) **Abstract**

A multi-chamber container for storing and applying eye drops. A container body (1) of the multi-chamber container has at least two chambers, i.e., a first chamber (3) and a second chamber (4) which are completely separated by means of a separable seal portion, wherein the first chamber (3) and the second chamber (4) respectively independently encapsulate different types of medicaments; and a weak weld (2) is provided between the first chamber (3) and the second chamber (4), so as to avoid changes over time when these agents are mixed together. The seal portion in the multi-chamber container can be separated upon application of an external force to one of the chambers of the container, such that the separately encapsulated different medicaments are mixed together in a sterile state, and a mixed solution that can be applied directly to the eye can be formed. The first chamber (3) is provided with an outflow port (5).

## Description

### TECHNICAL FIELD

The present disclosure relates to a container for storing and administering eye drops, and also relates to a method for manufacturing the multi-chamber container.

### BACKGROUND

Human eyes can tolerate drugs within a pH range of 5.0-9.0, and the pH of eye drops is usually roughly within this range. When at a pH of less than 5.0 or greater than 11.4, eye drops will cause significant irritation to the eyes. However, within the above tolerable pH range for human eyes, some common active ingredients in ophthalmic drugs exhibit instability due to susceptibility to hydrolysis, for example, drugs containing ester or amide bonds that are prone to hydrolysis. In particular, for children and adolescents, a high-risk group for myopia, ocular discomfort caused after medication often leads to poor compliance. In order to reduce the irritation to the eyes, the current commercially available products are still at a solution pH that is unfavorable for stable storage of the eye drops, making them unsuitable for extended storage. Containers with a plurality of chambers have been employed to store medically mixed solutions with time-dependent instability. One type of container utilizes a detachable seal to form an isolation structure. The detachable seal may be broken by tension or when pressure is applied to a liquid-filled container chamber adjacent thereto. The multi-chamber container with the detachable seal allows for a larger opening, facilitating full mixing of solutions. However, this type of container has only been reported in the form of flexible bags made of highly elastic and heat-resistant film materials (see CN1976668A). For such multi-chamber flexible infusion bags, several common issues remain to be addressed. First, the detachable seal has been improperly opened due to unavoidable external forces during transportation and the like before reaching a target user. Second, when the detachable seal is intact when reaching the target user, the detachable seal is hard to open, resulting in the failure to form a target mixed solution. It has been reported that for multi-chamber flexible medical infusion bags, the pull-open or roll-open methods are preferred for detaching the sealing part for content mixing. However, even specially trained clinical professionals or nursing staff cannot guarantee easy and safe opening of most flexible containers with a detachable sealing part. Consequently, ordinary users (particularly the elderly, children, and individuals with hand disabilities or reduced muscle strength, for example) generally cannot use the containers independently without the assistance of special tools. Third, the film material and the opening mode of the flexible bag render it unsuitable for containing a small-volume medical liquid, and its design is typically limited to a single-use application. Therefore, in the field of eye drops requiring drop-by-drop administration, currently, the commonly used containers are still single-chamber eye drops bottles made of plastic and other materials with certain rigidity.

In the prior art, there are also multi-chamber containers that employ isolation components made of rigid frangible materials to isolate chambers. While such multi-chamber containers have the advantage of high versatility, the isolation components thereof typically form only limited fracture cross-sections, which is not conducive to full mixing of solutions. Moreover, a significantly increased number of undesired particles are introduced into the mixed solution due to the damage of the isolation structure (see EP0378183). These increased insoluble particles are particularly associated with discomfort caused by the use of the ophthalmic solution and may even lead to non-compliance with mandatory criteria associated with eye drops.

### SUMMARY

An object of the present disclosure is to provide a container for eye drops which is provided with at least two chambers isolated from each other by a detachable seal and can be easily and reliably opened by a general user without special training and without assistance of any special tool, thereby not only ensuring the stability of the stored ophthalmic solutions at a low pH or a high pH, but also effectively avoiding discomfort caused by a non-physiological pH and insoluble particles irritating the human eyes.

In order to solve the above problems, the present disclosure provides a multi-chamber container for eye drops, where a container body is composed of two or more chambers including at least a first chamber and a second chamber, the first chamber is in communication with an outlet, the remaining chambers are connected to the first chamber or other chambers, and adjacent chambers are separated by a sealing part (2) and thus are not in communication with each other; application of an external force to an outer wall of one of two adjacent chambers causes the sealing part between the two adjacent chambers to be irreversibly detached, and when all the sealing parts are detached, each chamber becomes directly or indirectly in communication with the first chamber.

According to the above multi-chamber container of the present disclosure, a cap is further provided on the outlet, and the cap is either a one-time-opening cap or a cap capable of being opened and closed repeatedly.

According to the above multi-chamber container of the present disclosure, the container body is made of rigid plastic.

According to the above multi-chamber container of the present disclosure, the multi-chamber container is composed of two chambers.

According to the above multi-chamber container of the present disclosure, the sealing part is formed by weakly welding portions of the container body together using ultrasonic waves at an electric cylinder torque of 232-266 N and a welding time of 0.290-0.370 s.

According to the above multi-chamber container of the present disclosure, the sealing part is formed by weakly welding portions of the container body together using ultrasonic waves at an amplitude of 10%-42% and a welding time of 0.19-0.64 s, preferably at an amplitude of 36%-39% and a welding time of 0.47-0.49 s.

According to the above multi-chamber container of the present disclosure, the external force capable of resulting in irreversible detachment of the sealing part does not exceed an average pinch force of fingers of children aged 5-7 years, preferably not exceeding about 17-27 Newtons (N).

According to the above multi-chamber container of the present disclosure, the heat seal strength of the sealing part (2) is not less than 3 N, not less than 5 N, not less than 7 N, not less than 10 N, not less than 12 N, not less than 14 N, or not less than 16 N, and is not more than 62 N, not more than 60 N, not more than 58 N, not more than 55 N, not more than 51 N, not more than 45 N, not more than 40 N, not more than 38 N, not more than 34 N, or not more than 30 N, and is preferably 3-60 N, 5-58 N, 7-58 N, 10-55 N, 12-51 N, or 16-38 N.

As in different solutions, the heat seal strength of the sealing part (2) is not less than 3 N and not more than 62 N, not less than 3 N and not more than 60 N, not less than 3 N and not more than 58 N, not less than 3 N and not more than 55 N, not less than 3 N and not more than 51 N, not less than 3 N and not more than 45 N, not less than 3 N and not more than 40 N, not less than 3 N and not more than 38 N, not less than 3 N and not more than 34 N, or not less than 3 N and not more than 30 N. As in yet different solutions, the heat seal strength of the sealing part (2) is not less than 5 N and not more than 62 N, not less than 5 N and not more than 60 N, not less than 5 N and not more than 58 N, not less than 5 N and not more than 55 N, not less than 5 N and not more than 51 N, not less than 5 N and not more than 45 N, not less than 5 N and not more than 40 N, not less than 5 N and not more than 38 N, not less than 5 N and not more than 34 N, or not less than 5 N and not more than 30 N. As in yet different solutions, the heat seal strength of the sealing part (2) is not less than 7 N and not more than 62 N, not less than 7 N and not more than 60 N, not less than 7 N and not more than 58 N, not less than 7 N and not more than 55 N, not less than 7 N and not more than 51 N, not less than 7 N and not more than 45 N, not less than 7 N and not more than 40 N, not less than 7 N and not more than 38 N, not less than 7 N and not more than 34 N, or not less than 7 N and not more than 30 N. As in yet different solutions, the heat seal strength of the sealing part (2) is not less than 10 N and not more than 62 N, not less than 10 N and not more than 60 N, not less than 10 N and not more than 58 N, not less than 10 N and not more than 55 N, not less than 10 N and not more than 51 N, not less than 10 N and not more than 45 N, not less than 10 N and not more than 40 N, not less than 10 N and not more than 38 N, not less than 10 N and not more than 34 N, or not less than 10 N and not more than 30 N. As in yet different solutions, the heat seal strength of the sealing part (2) is not less than 12 N and not more than 62 N, not less than 12 N and not more than 60 N, not less than 12 N and not more than 58 N, not less than 12 N and not more than 55 N, not less than 12 N and not more than 51 N, not less than 12 N and not more than 45 N, not less than 12 N and not more than 40 N, not less than 12 N and not more than 38 N, not less than 12 N and not more than 34 N, or not less than 12 N and not more than 30 N. As in yet different solutions, the heat seal strength of the sealing part (2) is not less than 14 N and not more than 62 N, not less than 14 N and not more than 60 N, not less than 14 N and not more than 58 N, not less than 14 N and not more than 55 N, not less than 14 N and not more than 51 N, not less than 14 N and not more than 45 N, not less than 14 N and not more than 40 N, not less than 14 N and not more than 38 N, not less than 14 N and not more than 34 N, or not less than 14 N and not more than 30 N. As in yet different solutions, the heat seal strength of the sealing part (2) is not less than 16 N and not more than 62 N, not less than 16 N and not more than 60 N, not less than 16 N and not more than 58 N, not less than 16 N and not more than 55 N, not less than 16 N and not more than 51 N, not less than 16 N and not more than 45 N, not less than 16 N and not more than 40 N, not less than 16 N and not more than 38 N, not less than 16 N and not more than 34 N, or not less than 16 N and not more than 30 N.

According to the above multi-chamber container of the present disclosure, the container further includes a container tail (7). In some embodiments, the tail is formed under welded seal conditions of an air cylinder pressure of 2.0-2.5 bar and a welding time of 0.16-0.22 s. In other embodiments, the tail is formed under welded seal conditions of an ultrasonic amplitude not less than 43% and a welding time not less than 0.65 s. The tail is designed to form a closed end that is not likely to be broken for the above multi-chamber container of the present disclosure.

According to the above multi-chamber container of the present disclosure, the container body is tubular.

According to the above multi-chamber container of the present disclosure, the tubular container body has a length of 15-180 mm and an inner diameter of 4-30 mm.

According to the above multi-chamber container of the present disclosure, the thickness of the rigid plastic forming the container is 200-2000 µm, preferably 350-500 µm, and more preferably 380-400 µm.

According to the above multi-chamber container of the present disclosure, the container has a capacity not less than 0.2 mL and not more than 20 mL.

The present disclosure further provides an eye drops product with different medicaments separately filled in a plurality of chambers of the above multi-chamber container, where at least one of the medicaments has a pH value lower than about 5.3 or higher than about 8.3, a mixed solution formed by mixing the medicaments filled in the plurality of chambers has a pH value of about 5.3 to about 8.3, and the stability (e.g., hydrolytic stability) of at least one of the medicaments is higher than the stability of the mixed solution formed by mixing the medicaments filled in the different chambers.

The present disclosure further provides a method for manufacturing the above eye drops product. The method includes:
selecting a material suitable for forming a container body, where the material is a hollow structure with two or more open ends;
forming an outlet at an opening at one end of the hollow structure, and preferably providing a cap on the outlet, where the cap is either a one-time-opening cap or a cap capable of being opened and closed repeatedly, and the outlet is in communication with a first chamber;
with the outlet being in a closed state, filling a first specified volume of a first medicament into the hollow structure from an opening at another end;
performing weak welding at a position on the hollow structure corresponding to the first specified volume to form a detachable sealing part, thereby forming a sealed first chamber containing the first medicament between the detachable sealing part and the outlet;
continuing to fill a second specified volume of a second medicament from the opening at the another end into a second chamber formed between the opening and the detachable sealing part, where the second medicament is different from the first medicament;
carrying on in the same manner to optionally form more chambers that are filled with more different medicaments and separated from each other by the sealing parts; and
after filling a last medicament into a last chamber, forming a container tail by sealing the last chamber through welding;
in this way, the medicaments filled in the respective chambers are capable of eventually being mixed directly or indirectly with the medicament in the first chamber by detachment of the sealing parts, thereby being discharged via the outlet.

According to the method for manufacturing the eye drops product of the present disclosure, each sealing part is formed by weak welding using ultrasonic waves at an electric cylinder torque of 232-266 N and a welding time of 0.290-0.370 s, and the last chamber is sealed by welding using ultrasonic waves at an air cylinder pressure of 2.0-2.5 bar and a welding time of 0.16-0.22 s to form the container tail.

According to the method for manufacturing the eye drops product of the present disclosure, each sealing part (2) is formed by weak welding using ultrasonic waves at an amplitude of 10%-42% and a welding time of 0.19-0.64 s, preferably at an amplitude of 36%-39% and a welding time of 0.47-0.49 s, and the last chamber is sealed by welding under welded seal conditions of an amplitude not less than 43% and a welding time not less than 0.65 s to form the container tail (7).

According to the method for manufacturing the eye drops product of the present disclosure, the container body is made of rigid plastic.

According to the method for manufacturing the eye drops product of the present disclosure, the multi-chamber container is composed of two chambers.

According to the method for manufacturing the eye drops product of the present disclosure, an external force capable of resulting in irreversible detachment of the sealing part does not exceed an average pinch force of fingers of children aged 5- 7 years, preferably not exceeding about 17-27 Newtons (N).

According to the method for manufacturing the eye drops product of the present disclosure, the heat seal strength of the sealing part (2) is not less than 3 N, not less than 5 N, not less than 7 N, not less than 10 N, not less than 12 N, not less than 14 N, or not less than 16 N, and is not more than 62 N, not more than 60 N, not more than 58 N, not more than 55 N, not more than 51 N, not more than 45 N, not more than 40 N, not more than 38 N, not more than 34 N, or not more than 30 N, and is preferably 3-60 N, 5-58 N, 7-58 N, 10-55 N, 12-51 N, or 16-38 N. Specific ranges of the heat seal strength of the sealing part (2) are the same as those described above and will not be listed repeatedly.

According to the method for manufacturing the eye drops product of the present disclosure, the container body is tubular.

According to the method for manufacturing the eye drops product of the present disclosure, the tubular container body has a length of 15-180 mm and an inner diameter of 4-30 mm. According to the method for manufacturing the eye drops product of the present disclosure, the thickness of the rigid plastic is 200-2000 µm, preferably 350-500 µm, and more preferably 380-400 µm.

According to the method for manufacturing the eye drops product of the present disclosure, the multi-chamber container has a capacity not less than 0.2 mL and not more than 20 mL.

The multi-chamber container or the eye drops product of the present disclosure can achieve the following excellent effects.
1. Since the different chambers are independent of each other before use, they can provide relatively suitable storage environments for some drugs that are difficult to preserve before use. At the time of use, suitable conditions for use can be achieved for the final formulation through temporary mixing, thereby increasing medication compliance while extending the shelf life of the drug.
2. The weak welding structure of the present disclosure can stably separate different chambers, maintain structural stability under normal external forces during production, transportation, storage, sales, etc., and can be easily pinched open from the outside by users ranging from children to the elderly, without the assistance of special tools. The pinch-to-open process does not compromise the sealed environment inside the container, such that the sterile environment is ensured, and the very small number of insoluble particles produced after the pinch-to-open process do not compromise the quality of the drug.
3. The container of the present disclosure has a small overall volume with a total loading capacity of about not more than 20 mL, and is mainly used as a container for medical solutions with small usage volumes, such as eye drops. The loading capacity of the container of the present disclosure may be a single dose, or may meet requirements of multiple doses (typically not exceeding doses for a single month). The small dose and good sealing performance of the container of the present disclosure allow the container to meet relevant requirements of pharmaceutical products without adding bacteriostatic agents such as benzalkonium chloride. Therefore, the irritation of bacteriostatic substances to human organs, particularly sensitive organs such as eyeballs, is avoided.

### BRIEF DESCRIPTION OF THE DRAWINGS

FIG. 1 is an example of a multi-chamber container for eye drops of the present application, where
   in the figure, 1 represents a container body, 2 represents a weak-welding seam, 3 represents a first chamber, 4 represents a second chamber; 5 represents an outlet, 6 represents a cap, and 7 represents a container tail; and
FIG. 2 illustrates a sample for determining heat seal strength obtained from a dual-chamber container disclosed herein and a test for determining the heat seal strength, where
   in the figure, 8 represents an upper fixture, 9 represents a lower fixture, 10 represents a sheet obtained from the container body; 11 represents the width of the weak-welding seam, and 12 represents the length (W) of the weak-welding seam of the sample.

### DETAILED DESCRIPTION

The container of the present disclosure will be further described below with reference to the accompanying drawings and specific embodiments. The examples are given only for illustrating the present disclosure but not for limiting the scope of the present disclosure. The examples provided below can be used as a guide for further improvement by those of ordinary skill in the art, and are not intended to limit the present disclosure in any way. As used herein, the term "include" or "comprise" means that a structure or a process includes the recited components or steps, but does not exclude other components or steps. As used herein, the term "about" refers to a common error range for a corresponding value readily known to those skilled in the art. A value or parameter described herein by "about" includes the value or parameter itself.

The term "multi-chamber" or "a plurality of chambers" includes not only the case of two chambers exemplified in a specific example, but also the cases of more chambers, e.g., three or more chambers, that can be readily conceived by those skilled in the art based on the disclosure of the present disclosure and specific needs.

The term "rigid plastic" herein refers to a plastic that deforms due to an external force applied, for example, by finger pinching and automatically returns to its original shape after the external force is removed. For example, this plastic may be conventional plastic materials used for single-dose or multi-dose ophthalmic dropper bottles (see "General Chapter 9621 Guidelines for General Requirements for Drug Packaging Materials and Containers" in Pharmacopoeia of the People's Republic of China (2015), Volume IV; and National Standards for Pharmaceutical Packaging Materials, compiled by the National Institutes for Food and Drug Control, published by China Medical Science Press). In other words, the plastic used to manufacture the container of the present disclosure may be any plastic known in the art to be suitable for manufacturing single-dose or multi-dose pharmaceutical solution bottles, as long as it has an elastic modulus that enables the reversible deformation and recovery required by the present disclosure. As a non-limiting example, the rigid plastic used in the present disclosure may be, for example, polyolefin resins, including but not limited to, polyethylene-based resins such as low-density polyethylene, polypropylene-based resins such as polypropylene, cyclic polyolefin resins, and composite laminate materials including different polyolefins described above. For the rigid plastic used in the present disclosure, reference may also be made to, for example, Quick Reference Manual for Common Plastics, edited by Qi Guiliang, *et al.* As a non-limiting example of the "rigid plastic" in the present disclosure, reference may also be made to examples described in the patent application entitled "LOW-CONCENTRATION ATROPINE DRUG PRODUCT AND MANUFACTURING METHOD THEREOF" filed by the applicant on the same day as the filing date of the present application. The application filed on the same day is incorporated herein by reference in its entirety.

In one aspect of the present disclosure, the rigid plastic used in the container of the present disclosure has a thickness that not only allows for the reversible deformation and recovery required by the container of the present disclosure, but also provides the mechanical strength required by a pharmaceutical container, in particular an eye drops container. In a preferred embodiment, the rigid plastic used in the container of the present disclosure has a thickness of 200-2000 µm, preferably 350-500 µm, and more preferably 380-400 µm. When it is necessary to block oxygen, water vapor, light, etc., the resin used in the container of the present disclosure may also be laminated with a metal material such as aluminum foil. In this case, the thickness of the rigid plastic in the container of the present disclosure is a thickness after all the laminated materials are included.

In one aspect of the present disclosure, the container of the present disclosure may be of any suitable shape as long as it is suitable for containing a single dose or multiple doses of a pharmaceutical solution. In a preferred embodiment, the container of the present disclosure is made of tubular rigid plastic having a certain length and diameter. Herein, "tubular" refers to a hollow columnar structure having a certain length. In some embodiments, the length of the tubular structure is significantly greater than its diameter. In a preferred embodiment, the tubular structure has a length of 15-180 mm and an inner diameter of 4-30 mm.

In one aspect of the present disclosure, the container of the present disclosure has a capacity suitable for containing a single dose or multiple doses of a pharmaceutical solution. Herein, the "capacity" refers to the sum of volumes of solutions that can be contained by all chambers included in the container. In some embodiments, the container of the present disclosure has a capacity not less than 0.2 mL and not more than 20 mL.

An approximate correspondence between capacity and tubing dimensions is as follows:

**Table 1. Correspondence between capacity and tubing dimensions**

| Capacity | Length | Diameter |
|---|---|---|
| 0.2 mL-5 mL | 30-100 mm | 4 mm-15 mm |
| 5 mL-10 mL | 50 mm-120 mm | 7 mm-20 mm |
| 10 mL-15 mL | 70 mm-140 mm | 10 mm-25 mm |
| 15 mL-20 mL | 90 mm-160 mm | 13 mm-30 mm |

In one aspect of the present disclosure, the terms "detachable sealing component" and "weak-welding seam" are used interchangeably and refer to a structure primarily resulting from the container body of the present disclosure undergoing a weak welding process. The structure can isolate different chambers of the container of the present disclosure, and can peel off or detach under a certain force. The weak-welding seam of the container of the present disclosure may adopt any design, as long as it can easily detach due to pressure applied on any chamber wall by a user for the purpose of use but will not suffer from undesired detachment due to normal external forces during preparation, packaging, transportation, sales, etc. In some embodiments, the weak-welding seam may be in an elongated linear structure. In other embodiments, the weak-welding seam may also be in a curved arcuate structure or in a line-intersecting structure with an included angle. In some embodiments, the weak-welding seam has a uniform equal width. In other embodiments, the weak-welding seam has a variable width. In a preferred embodiment, the width of the weak-welding seam is 1-20 mm, preferably 2-8 mm, and more preferably 4 mm. The term "width", when describing the sealing component, refers to the length occupied by the component in the direction of the long axis of the container.

In one aspect of the present disclosure, the plurality of chambers of the container of the present disclosure are separately filled with different medical solutions. For example, in the case that the container of the present disclosure is provided with two chambers, a first chamber may be filled with a drug prone to hydrolysis, and a second chamber may be filled with an aqueous solution of pharmaceutically acceptable excipient ingredients. As a non-limiting example, the drug that may be filled in the first chamber may be ester drugs such as tetracaine hydrochloride, cocaine hydrochloride, propantheline bromide, atropine sulfate, or homatropine hydrobromide, amide drugs, or the like. Non-limiting examples of the drug that may be filled in the second chamber include, but are not limited to, buffer salts, osmotic pressure regulators, preservatives, and/or metal ion chelating agents, or the like. It will be understood by those skilled in the art that the contents in the chambers of the container of the present disclosure may be changed. For example, the solutions in the two chambers may be separately replaced with other suitable solutions, as long as the solutions can be eventually mixed to form eye drops that can be directly administered to the eyes. For example, the ester drug in the first chamber may be replaced with a lactone drug such as pilocarpine nitrate or warfarin sodium. In other aspects of the present disclosure, the assignment of the filling solutions to the chambers of the container may also be varied, i.e., an aqueous solution of a pharmaceutically acceptable excipient such as a buffer may be contained in the first chamber, while a hydrolysis-prone drug solution may be contained in the second chamber. In other aspects of the present disclosure, the user should uniformly mix these two solutions together immediately before administration, so as to form eye drops for direct administration to the eyes.

In one aspect of the present disclosure, the outlet and the cap used in the container of the present disclosure may adopt any outlet and cap structures conventionally used in the art, as long as the solution in the container can be extruded from the container in a dropwise manner when the cap is opened. In other aspects of the present disclosure, the outlet of the container of the present disclosure may also be a closed port without capping, as long as it can be easily opened for use. In other aspects of the present disclosure, the outlet of the container of the present disclosure may also be a cap that can be repeatedly opened and closed and is connected to or separated from the body of the multi-chamber container.

In one aspect of the present disclosure, the position for weak welding may be readily determined based on the amount of the target solutions injected. For example, when the two target solutions injected are of equal volume, weak welding is performed approximately at the midpoint of the container body. In a specific embodiment, for the process for weak welding of the container of the present disclosure, ultrasonic welding is preferably adopted, although other suitable welding methods may also be selected. As a non-limiting example of weak welding, an ultrasonic welding machine (model: STD20-1500W) equipped with an electric actuator (model: LEYH32NZC-50M) or an ultrasonic welding machine equipped with an ultrasonic generator (model: EDG-2020) may be used for performing weak welding.

In one aspect of the present disclosure, the process for the welded seal of the tail is a process commonly used in the art for welding plastic materials, including but not limited to thermal welding, ultrasonic welding, and the like. In a preferred aspect of the present disclosure, ultrasonic welding is used for tail sealing of the container of the present disclosure. As a non-limiting example of the welded seal of tail, an ultrasonic welding machine (model: STD-2000W) equipped with an air cylinder (model: MD81840-50Z) or an ultrasonic welding machine equipped with an ultrasonic generator (model: EDG-2020) may be used for performing tail sealing.

In the following examples, similar or identical components will be denoted by the same reference numerals and will not be described repeatedly.

### Example 1: Structure, Composition, and Usage of Container

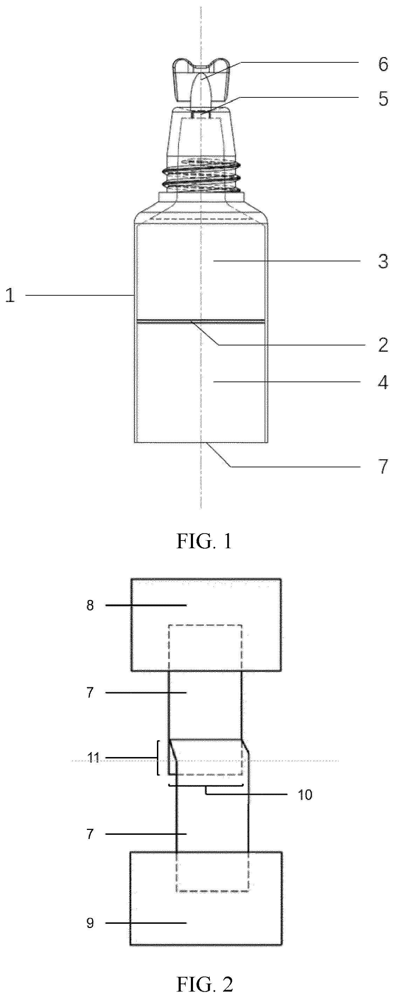

FIG. 1 shows a specific example of the present application.

A body 1 of a multi-chamber container includes a first chamber 3 and a second chamber 4. A sealing part 2 (i.e., a weak-welding seam) is formed between the first chamber 3 and the second chamber 4.

The container body 1 is made of polyolefin resin, which allows for weak welding using an ultrasonic process and provides transparency, hygienic properties, and appropriate elasticity and rigidity.

During drug administration, without requiring any specific auxiliary device, the weak-welding seam can be opened only by pressing the first chamber 3 or the second chamber 4, for example, by manually pressing with one hand, to increase the internal pressure of the chamber, such that the first chamber 3 and the second chamber 4 can be in communication with each other and the medicament solutions stored in the two chambers are mixed together. Moreover, manual pressure application will not damage a tail 7 of the multi-chamber container, thereby preventing undesirable leakage of the content of the container from the tail. Subsequently, a cap 6 at the front end of an outlet 5 is twisted off to expose the outlet 5, allowing the formed mixed solution to be extruded dropwise through the outlet 5.

### Example 2: Manufacture of Multi-Chamber Eye Drops Product

The multi-chamber eye drops product of the present disclosure may be manufactured by using the following process.

A body of a dual-chamber container was manufactured using a low-density polyethylene (LDPE) flexible tube with a diameter of 13.0 mm (±0.2 mm) and with two open ends.

Firstly, the polyolefin flexible tube was cut into short tubes of a uniform length, and the specific length may be appropriately adjusted according to the intended amount of drug to be loaded, which is generally not less than about 40 mm. In this example, the flexible tube was cut into short tubes with a length of 40 mm.

Then, an outlet 5 was formed at one end of the short tube by using a conventional blow-molding process, where the outlet was in communication with the chamber in the container body. The container was further provided with a cap 6 adapted to the outlet 5 for closing the outlet.

A first medicament solution was injected into the container body 1 from the other end of the flexible tube without the outlet. Then, ultrasonic weak welding was performed at the midpoint of the length of the container body 1 by using an ultrasonic welding machine (Model: STD20-1500W) equipped with an electric actuator (model: LEYH32NZC-50M) for 0.290-0.370 s. After weak welding, two chambers not in communication were formed in the container body 1, namely, a first chamber 3 with the outlet 5 and a second chamber 4 that is away from the outlet 5 and connected to the first chamber 3 through weak welding, where the first chamber 3 has been filled with the first medicament solution, and the second chamber was a hollow structure containing no solution.

A second medicament solution was filled into the container body 1 from the opening of the hollow structure described above, and then the opening was closed by welding using an ultrasonic welding machine (model: STD-2000W) equipped with an air cylinder (model: MD81840-50Z) to form a container tail 7, thereby forming a closed second chamber 4 filled with the second medicament solution.

In this example, the complete sealing rate was tested for cases where the container tails were formed by welded seal under different manufacture parameters of an air cylinder pressure of 1.7-2.5 bar and a welding time of 0.16-0.22 s. The results are shown in Table 2 below.

**Table 2. Parameters for forming the tail by welded seal**

| Group (n=50) | Welding time (s) | Air cylinder pressure (bar) | Complete sealing rate (%) |
|---|---|---|---|
| 1 | 0.19 | 1.7 | 70 |
| 2 | 0.16 | 2.0 | 98 |
| 3 | 0.19 | 2.2 | 100 |
| 4 | 0.22 | 2.5 | 100 |

In this example, the complete sealing rate and the pinch-to-open rate were tested for cases where the weak-welding seams of the container were formed by welded seal under different manufacture parameters of an electric cylinder torque of 232-266 N and a welding time of 0.200-0.370 s. The results measured by using conventional instruments are shown in Table 3 below.

**Table 3. Parameters for forming the weak-welding seam**

| Group (n=20) | Welding time (s) | Electric cylinder torque (N) | Complete sealing rate (%) | Pinch-to-open rate (%) |
|---|---|---|---|---|
| 1 | 0.200 | 237 | 55 | Not determined |
| 2 | 0.295 | 266 | 90 | 85 |
| 3 | 0.370 | 237 | 100 | 75 |
| 4 | 0.500 | 266 | 100 | 10 |
| 5 | 0.270 | 266 | 75 | Not determined |
| 6 | 0.295 | 266 | 90 | 85 |
| 7 | 0.305 | 266 | 100 | 85 |
| 8 | 0.370 | 266 | 100 | 45 |
| 9 | 0.305 | 237 | 90 | 100 |
| 10 | 0.305 | 241 | 100 | 100 |
| 11 | 0.305 | 266 | 100 | 85 |
| 12 | 0.270 | 237 | 70 | Not determined |
| 13 | 0.295 | 237 | 85 | 100 |
| 14 | 0.345 | 237 | 100 | 95 |
| 15 | 0.370 | 237 | 100 | 75 |
| 16 | 0.345 | 232 | 90 | 100 |
| 17 | 0.345 | 234.8 | 95 | 100 |
| 18 | 0.345 | 238.6 | 100 | 100 |
| 19 | 0.345 | 237 | 100 | 95 |

Under simulated daily use conditions, 15 eye drops bottles were independently pinched open by each of six children (mean age: 7 years), six young adults (mean age: 28 years), and six elderly adults (mean age: 60 years). An average time required for each subject to fully open the weak-welding seam of the eye drops bottle by pinching was measured and shown in Table 4 below. In the table, Group A represents weak welding at a cylinder torque of 238.6 N for 0.345 s, and Group B represents weak welding at a cylinder torque of 241 N for 0.305 s.

**Table 4. Pinch-to-open test**

| Group | Child A (n=15) | Child B (n=15) | Child C (n=15) | Child D (n=15) | Child E (n=15) | Child F (n=15) |
|---|---|---|---|---|---|---|
| Group A | 6s | 6s | 5 s | 6s | 5 s | 7 s |
| Group B | 3 s | 4s | 3 s | 4s | 3 s | 4s |
| | Young adult A (n=15) | Young adult B (n=15) | Young adult C (n=15) | Young adult D (n=15) | Young adult E (n=15) | Young adult F (n=15) |
| Group A | 3 s | 3 s | 3 s | 3 s | 4s | 3 s |
| Group B | 1 s | 1 s | 2s | 1 s | 2s | 1 s |
| | Elderly adult A (n=15) | Elderly adult B (n=15) | Elderly adult C (n=15) | Elderly adult D (n=15) | Elderly adult E (n=15) | Elderly adult F (n=15) |
| Group A | 4s | 5 s | 4s | 4s | 5 s | 5 s |
| Group B | 2s | 2s | 2s | 2s | 3 s | 2s |

As can be seen from Table 4, changing the conditions of weak welding affects, to some extent, the time required for the subject to complete pinch-to-open operation of the container of the present disclosure. Overall, however, the weak-welding seam of the dual-chamber container of the present disclosure can be easily opened by general users without special training to facilitate subsequent eye drops administration. Moreover, even the children and elderly with relatively weak fine motor skills and pinch force can easily and quickly accomplish the pinch-to-open operation of the container of the present disclosure.

### Example 3: Manufacture of Multi-Chamber Eye Drops Product

The multi-chamber eye drops product of the present disclosure may also be manufactured by using the following process.

Following the same steps as in Example 2, the polyolefin flexible tube was subjected to outlet formation, first solution filling, weak welding, second solution filling, and tail sealing in sequence. Unlike Example 2, the polyolefin flexible tube used in this example was a polypropylene flexible tube, and the weak welding and the welded seal of the tail were completed by using the same ultrasonic welding device by varying the amplitude and welding time of the ultrasonic generator (model: EDG-2020).

In this example, the complete sealing rate was tested for cases where the tails of containers were formed by welded seal under different manufacture parameters of an amplitude of 10%-45% and a welding time of 0.19-0.95 s. The results are shown in Table 5 below.

**Table 5. Parameters for forming the tail by welded seal**

| Group | Welding time (s) | Amplitude (%) | Complete sealing rate (%) |
|---|---|---|---|
| 1 | 0.19 | 10 | 70 |
| 2 | 0.28 | 43 | 90 |
| 3 | 0.65 | 43 | 100 |
| 4 | 0.95 | 45 | 100 |

In this example, the complete sealing rate and the pinch-to-open rate were tested for cases where the weak-welding seams of the container were formed by welded seal under different manufacture parameters of an amplitude of 10%-45% and a welding time of 0.19-1.10 s. The results measured by using conventional instruments are shown in Table 6 below.

**Table 6. Parameters for forming the weak-welding seam**

| Group | Welding time (s) | Amplitude (%) | Complete sealing rate (%) | Pinch-to-open rate (%) |
|---|---|---|---|---|
| 1 | 0.19 | 10 | 55 | Not determined |
| 2 | 0.52 | 36 | 100 | 75 |
| 3 | 0.53 | 34 | 100 | 85 |
| 4 | 1.10 | 45 | 100 | 45 |
| 5 | 0.43 | 34 | 35 | Not determined |
| 6 | 0.46 | 34 | 65 | Not determined |
| 7 | 0.49 | 34 | 90 | Not determined |
| 8 | 0.53 | 34 | 100 | 85 |
| 9 | *0.55* | 34 | 100 | 45 |
| 10 | 0.42 | 36 | 45 | Not determined |
| 11 | 0.45 | 36 | 60 | Not determined |
| 12 | 0.47 | 36 | 85 | Not determined |
| 13 | 0.52 | 36 | 100 | 75 |
| 14 | 0.55 | 36 | 100 | 65 |
| 15 | 0.49 | 32 | 60 | Not determined |
| 16 | 0.49 | 34 | 90 | Not determined |
| 17 | 0.49 | 36 | 100 | 100 |
| 18 | 0.49 | 38 | 100 | 85 |
| 19 | 0.49 | 40 | 100 | 70 |
| 20 | 0.47 | 32 | 55 | Not determined |
| 21 | 0.47 | 36 | 85 | Not determined |
| 22 | 0.47 | 39 | 100 | 95 |
| 23 | 0.47 | 42 | 100 | 80 |
| 24 | 0.47 | 45 | 100 | 55 |

Under simulated daily use conditions, 15 eye drops bottles were independently pinched open by each of six children (mean age: 7 years), six young adults (mean age: 28 years), and six elderly adults (mean age: 60 years). An average time required for each subject to fully open the weak-welding seam of the eye drops bottle by pinching was measured and shown in Table 7 below. In the table, Group A represents weak welding at an amplitude of 39% for 0.47 s, and Group B represents weak welding at an amplitude of 36% for 0.49 s.

**Table 7. Pinch-to-open test**

| Group | Child A (n=15) | Child B (n=15) | Child C (n=15) | Child D (n=15) | Child E (n=15) | Child F (n=15) |
|---|---|---|---|---|---|---|
| Group A | 7s | 6s | 7s | 7s | 6s | 5 s |
| Group B | 4s | 3 s | 4s | 4s | 2s | 3 s |
| | Young adult A (n=15) | Young adult B (n=15) | Young adult C (n=15) | Young adult D (n=15) | Young adult E (n=15) | Young adult F (n=15) |
| Group A | 3 s | 4 s | 3 s | 4 s | 3 s | 3 s |
| Group B | 2 s | 1 s | 2 s | 1 s | 2 s | 1 s |
| | Elderly adult A (n=15) | Elderly adult B (n=15) | Elderly adult C (n=15) | Elderly adult D (n=15) | Elderly adult E (n=15) | Elderly adult F (n=15) |
| Group A | 4 s | 5 s | 4 s | 4 s | 5 s | 4 s |
| Group B | 2 s | 3 s | 2 s | 3 s | 3 s | 3 s |

As can be seen from Table 7, changing the conditions of weak welding affects, to some extent, the time required for the subject to complete pinch-to-open operation of the container of the present disclosure. Overall, however, the weak-welding seam of the dual-chamber container of the present disclosure can be easily opened by general users without special training.

### Example 4: Determination of Insoluble Particles

As an example of drug solutions stored in two chambers, in the present disclosure, the following drug solution compositions were used for measuring the insoluble particles in the mixed solution after the weak-welding seam was pinched open.

### Composition 1:

### First solution:

0.01%-0.05% of tetracaine hydrochloride, cocaine hydrochloride, propantheline bromide, etc.;
0.01%-0.09% of sodium dihydrogen phosphate;
0.01%-0.45% of disodium hydrogen phosphate;
0-0.9% of sodium chloride; and
the balance being water.

The pH was adjusted to 3.5-4.5.

### Second solution:

0-0.09% of sodium dihydrogen phosphate;
0-0.45% of disodium hydrogen phosphate;
0-0.9% of sodium chloride; and
0-0.1% of edetate disodium.

The pH was adjusted to 7.0-8.5.

### Composition 2:

### First solution:

pilocarpine nitrate 1%-2%, warfarin sodium, etc.;
0.1%-0.20% of sodium citrate hydrate;
0.5%-1.0% of boric acid;
0-0.9% of sodium chloride; and
the balance being water.

The pH was adjusted to 3-4.

### Second solution:

0.5%-1.0% of sodium citrate hydrate;
0-0.9% of sodium chloride; and
0-0.1% of benzalkonium chloride.

The pH was adjusted to 7-8.

In accordance with the method for measuring insoluble particles specified in *Pharmacopoeia of the People's Republic of China,* the number of insoluble particles was separately measured for purified water, an ophthalmic solution stored in a single-chamber container manufactured by a tail sealing process, and an ophthalmic solution stored in a dual-chamber container manufactured by the preferred process of the present disclosure (weak welding using the ultrasonic welding machine (model: STD20-1500W) equipped with the electric actuator (model: LEYH32NZC-50M) for 0.305 s at an electric cylinder torque of 241 N). The measurement results are shown in Table 8 below.

**Table 8. Determination results of insoluble particles**

| Item | ≥10 µm | ≥25 µm | Quality criteria (the number of particles of 10 µm or greater shall not exceed 6000, and the number of particles of 25 µm or greater shall not exceed 600) |
|---|---|---|---|
| Purified water | 14.5 | 0.4 | Compliant |
| | 11.6 | 0.3 | |
| | 15.2 | 0.6 | |
| | 12.3 | 0.5 | |
| | 13.2 | 0.1 | |
| Tail sealing only | 11.5 | 1.3 | Compliant |
| | 13.4 | 0.8 | |
| | 13.8 | 0.5 | |
| | 14.2 | 1.0 | |
| | 10.3 | 0.5 | |
| Tail sealing + Weak welding | 43.8 | 0.8 | Compliant |
| | 42.1 | 0.5 | |
| | 40.5 | 1.2 | |
| | 39.9 | 0.4 | |
| | 40.1 | 0.6 | |

The results show that the process of the present disclosure primarily affects the number of insoluble particles of 10 µm or greater in the solution contained in the container, with no statistically significant impact on the content of the particles of 25 µm or greater in the solution. Moreover, even for the particles of 10 µm or greater, the container manufactured using the process of the present disclosure still complies with the mandatory provisions of *Pharmacopoeia of the People's Republic of China* and other regulations.

### Example 5: Method for Determining Heat Seal Strength

This example describes a method for determining the heat seal strength of the dual-chamber eye drops bottle manufactured by using the method disclosed herein with reference to the "Determination of heat seal strength" section in China National Standard GB/T22638.7-2016 (which is incorporated herein by reference in its entirety). The method includes the following: The outlet and/or the tail were removed from the multi-chamber container to form an opening at one side of the container, two cutting positions were selected on the opening and the wall of the container was cut in a direction parallel to the longitudinal axis of the container to a position where the sealing component is located, thereby forming two strips at this side of the container body. Then, the container body at the other side was removed while precisely preserving the complete sealing component, ultimately obtaining a test sample consisting of the complete sealing component and the two strips. Then, as shown in FIG. 2, the two strips were respectively clamped in upper and lower fixtures of a tensile machine to measure the heat seal strength. During the measurement, one fixture was immobilized and the other was pulled at a constant speed (100 mm/min) until the sealing component was completely peeled off. The maximum tensile force recorded in this process was denoted as Fmax. The selection of the cutting positions may be readily determined based on the structure and orientation of the weak-welding seam. Specifically, the cutting positions were determined to be positions corresponding to two end points of the long axis of the weak-welding seam. That is, the lines connecting the cutting positions and the corresponding end points were parallel to the long axis of the container.

Taking a dual-chamber container with a bottle body length of 6.5 cm (excluding the cap) and an inner diameter of 1.2 cm and having a weak-welding seam of 4 mm (width) × 15 mm (length) as an example, the test sample obtained by using the method described above included a complete weak-welding seam (4 mm × 15 mm) in a sealed state and two strips (cut from the container body, each with a length of about 25 mm) at one side of the weak-welding seam. The tensile machine used in the test was a universal laboratory testing machine (model: C610M) purchased from Jinan Labthink Instruments Co., Ltd., without being limited thereto.

The heat seal strength (R) is calculated according to the following formula: R = Fmax/A, where Fmax is the maximum tensile force measured by the method described above, which may also be referred to as the maximum peel load and is expressed in Newtons (N); A is a length factor, corrected to A = W/15 mm, where W is the actual length (as shown in FIG. 2) of the weak-welding seam present on the test sample and is expressed in millimeters. In the case of the above example test sample, W is 15 mm.

### Example 6: Determination of Heat Seal Strength

The dual-chamber containers for eye drops disclosed herein were prepared from two types of medical-grade polyethylene (LDPE) tubings (1810E and 3020D) by using the method described in Example 2. The resulting containers had a length of about 6.5 cm (excluding the cap), with the width of the weak-welding seam being about 4 mm and the length of the weak-welding seam being about 10 mm. During ultrasonic weak welding for forming the sealing component, upper and lower limits of weak welding parameters were separately used to obtain test sample groups 1 in which the containers were just completely sealed and test sample groups 2 in which the containers could be barely pinched open by manual force (verified by adult males by repeating the pinch-to-open test described in Table 4).

Twenty samples were randomly selected from each of four groups of test samples, for which the heat seal strength of the weak-welding seam was measured by using the method described in Example 5. The results are shown in Table 9.

**Table 9. Determination of heat seal strength**

| Packaging material | 1810E | | 3020D | |
|---|---|---|---|---|
| | Group 1 | Group 2 | Group 1 | Group 2 |
| 1 | 11.642 | 51.143 | 5.392 | 40.173 |
| 2 | 13.587 | 47.913 | 4.836 | 41.633 |
| 3 | 14.069 | 51.580 | 3.27 | 40.966 |
| 4 | 13.905 | 44.508 | 3.791 | 51.558 |
| 5 | 14.628 | 58.284 | 2.84 | 39.987 |
| 6 | 11.040 | 44.055 | 6.999 | 45.332 |
| 7 | 13.249 | 49.432 | 5.108 | 40.041 |
| 8 | 8.775 | 42.805 | 6.118 | 39.915 |
| 9 | 13.206 | 44.679 | 5.493 | 39.753 |
| 10 | 13.414 | 44.702 | 3.496 | 40.042 |
| 11 | 7.475 | 50.397 | 3.864 | 42.385 |
| 12 | 13.932 | 43.661 | 4.153 | 46.648 |
| 13 | 12.353 | 43.6 | 4.41 | 45.575 |
| 14 | 13.547 | 43.394 | 6.937 | 51.332 |
| 15 | 13.301 | 56.102 | 3.895 | 42.759 |
| 16 | 13.256 | 54.372 | 6.898 | 39.689 |
| 17 | 11.777 | 55.537 | 4.56 | 41.876 |
| 18 | 14.370 | 42.8 | 5.414 | 40.599 |
| 19 | 13.074 | 42.727 | 4.721 | 47.031 |
| 20 | 9.677 | 58.077 | 4.403 | 42.651 |
| MAX | 14.628 | 58.284 | 6.999 | 51.558 |
| MIN | 7.475 | 42.727 | 2.840 | 39.689 |
| AVE | 12.514 | 48.488 | 4.830 | 42.997 |
| STDEV | 1.929 | 5.558 | 1.219 | 3.724 |
| RSD | 15.418 | 11.463 | 25.231 | 8.661 |

As can be seen from Table 9, for the multi-chamber container disclosed herein, the sealing part of the container can achieve complete isolation between adjacent chambers while allowing a user to pinch to open without tools when the heat seal strength of the weak-welding seam is within the following ranges: not less than 3 N, not less than 5 N, not less than 7 N, not less than 10 N, not less than 12 N, not less than 14 N, or not less than 16 N, and not more than 62 N, not more than 60 N, not more than 58 N, not more than 55 N, not more than 51 N, not more than 45 N, not more than 40 N, not more than 38 N, not more than 34 N, or not more than 30 N, and preferably 3-60 N, 5-58 N, 7-58 N, 10-55 N, 12-51 N, or 16-38 N.

### Example 7

Five samples were randomly selected from the test samples manufactured in Example 6, and the content of insoluble particles in the eye drops after the sealing parts were opened was determined by using the method described in Example 4. The results are shown in Table 10.

**Table 10.**

| Item | ≥10 µm | ≥25 µm | Quality criteria (the number of particles of 10 µm or greater shall not exceed 6000, and the number of particles of 25 µm or greater shall not exceed 600) |
|---|---|---|---|
| 1810E | 39.5 | 0.8 | Compliant |
| | 40.7 | 0.4 | |
| | 38.2 | 0.7 | |
| | 40.0 | 0.2 | |
| | 41.3 | 0.3 | |
| 3020D | 37.2 | 1.8 | Compliant |
| | 41.1 | 0.4 | |
| | 39.4 | 1.0 | |
| | 37.0 | 0.6 | |
| | 36.8 | 0.9 | |

As shown in Table 10, for the multi-chamber containers prepared from the two types of polyethylene tubings, the number of insoluble particles generated after opening the sealing component was only about 0.7% of the upper limit specified in *Pharmacopoeia of the People's Republic of China,* indicating that the container disclosed herein is suitable for use as an eye drops container, and that the operation of opening the sealing component does not cause a significant increase in the number of particles that may cause foreign body sensation.

The present disclosure has been described in detail above. For those skilled in the art, the present disclosure can be practiced over a relatively broad range using equivalent parameters, concentrations, and conditions without departing from the spirit and scope of the present disclosure and without the need for undue experimentation. While particular examples have been provided in the present disclosure, it should be understood that further improvements can be made to the present disclosure. In summary, in accordance with the principles of the present disclosure, the present application is intended to encompass any variations, uses, or improvements of the present disclosure, including modifications that depart from the disclosed scope of the present application but are made by using conventional techniques known in the art. Certain essential features may be utilized within the scope of the appended claims.

## Claims

1. A multi-chamber container for eye drops, wherein a container body (1) is composed of two or more medicament-storing chambers comprising at least a first chamber (3) and a second chamber (4), the first chamber (3) is in communication with an outlet (5), the remaining chambers are connected to the first chamber or other chambers, and adjacent chambers are separated by a sealing part (2) and thus are not in communication with each other; application of an external force to an outer wall of one of two adjacent chambers causes the sealing part between the two adjacent chambers to be irreversibly detached, and when all the sealing parts are detached, each chamber becomes directly or indirectly connected to the first chamber.

2. The multi-chamber container according to claim 1, wherein a cap (6) is further provided on the outlet (5), and the cap (6) is either a one-time-opening cap or a cap capable of being opened and closed repeatedly.

3. The multi-chamber container according to any one of claims 1-2, wherein the container body is made of rigid plastic, and preferably, a thickness of the rigid plastic is 200-2000 µm, preferably 350-500 µm, and more preferably 380-400 µm.

4. The multi-chamber container according to any one of claims 1-3, wherein the multi-chamber container is composed of two chambers.

5. The multi-chamber container according to any one of claims 1-4, wherein the sealing part (2) is formed by weakly welding portions of the container body together using ultrasonic waves at an electric cylinder torque of 232-266 N and a welding time of 0.290-0.370 s; optionally, the sealing part (2) is formed by weakly welding portions of the container body together using ultrasonic waves at an amplitude of 10%-42% and a welding time of 0.19-0.64 s, preferably at an amplitude of 36%-39% and a welding time of 0.47-0.49 s.

6. The multi-chamber container according to any one of claims 1-5, wherein the external force capable of resulting in irreversible detachment of the sealing part does not exceed an average pinch force of fingers of children aged 5-7 years, preferably not exceeding 17-27 Newtons (N).

7. The multi-chamber container according to any one of claims 1-6, wherein a heat seal strength of the sealing part (2) is not less than 3 N, not less than 5 N, not less than 7 N, not less than 10 N, not less than 12 N, not less than 14 N, or not less than 16 N, and is not more than 62 N, not more than 60 N, not more than 58 N, not more than 55 N, not more than 51 N, not more than 45 N, not more than 40 N, not more than 38 N, not more than 34 N, or not more than 30 N, and is preferably 3-60 N, 5-58 N, 7-58 N, 10-55 N, 12-51 N, or 16-38 N.

8. The multi-chamber container according to any one of claims 1-7, wherein the container further comprises a container tail (7), and the tail is formed under welded seal conditions of an air cylinder pressure of 2.0-2.5 bar and a welding time of 0.16-0.22 s;
optionally, the container further comprises a container tail (7), and the tail is formed under welded seal conditions of an ultrasonic amplitude not less than 43% and a welding time not less than 0.65 s.

9. The multi-chamber container according to any one of claims 1-8, wherein the container body is tubular; preferably, the tubular container body has a length of 15-180 mm and an inner diameter of 4-30 mm.

10. The multi-chamber container according to any one of claims 1-9, wherein the multi-chamber container has a capacity not less than 0.2 mL and not more than 20 mL.

11. The multi-chamber container according to any one of claims 1-10, wherein the sealing part is in an elongated linear structure, or in a curved arcuate structure, or in a line-intersecting structure with an included angle; optionally, the sealing part has a uniform equal width or a variable width, and preferably, the width of the sealing part is 1-20 mm, preferably 2-8 mm, and more preferably 4 mm.

12. An eye drops eye drops product with different medicaments separately filled in a plurality of chambers of the multi-chamber container according to any one of claims 1-11, wherein at least one of the medicaments has a pH value lower than 5.3 or higher than 8.3, a mixed solution formed by mixing the medicaments filled in the plurality of chambers has a pH value of 5.3-8.3, and stability of at least one of the medicaments is higher than stability of the mixed solution formed by mixing the medicaments filled in the different chambers.

13. A method for manufacturing the eye drops product according to claim 12, comprising:
selecting a material suitable for forming a container body (1), wherein the material is a hollow structure with two or more open ends;
forming an outlet (5) at an opening at one end of the hollow structure, and preferably providing a cap (6) on the outlet (5), wherein the cap (6) is either a one-time-opening cap or a cap capable of being opened and closed repeatedly, and the outlet (5) is in communication with a first chamber (3);
with the outlet (5) being in a closed state, filling a first specified volume of a first medicament into the hollow structure from an opening at another end;
performing weak welding at a position on the hollow structure corresponding to the first specified volume to form a detachable sealing part (2), thereby forming a sealed first chamber (3) containing the first medicament between the detachable sealing part (2) and the outlet (5);
continuing to fill a second specified volume of a second medicament from the opening at the another end into a second chamber (4) formed between the opening and the detachable sealing part (2), wherein the second medicament is different from the first medicament;
carrying on in the same manner to optionally form more chambers that are filled with more different medicaments and separated from each other by the sealing parts; and
after filling a last medicament into a last chamber, forming a container tail (7) by sealing the last chamber through welding,
wherein in this way, the medicaments filled in the respective chambers are capable of eventually being mixed directly or indirectly with the medicament in the first chamber (3) by detachment of the sealing parts, thereby being discharged via the outlet (5).

14. The method for manufacturing the eye drops product according to claim 13, wherein each sealing part (2) is formed by weak welding using ultrasonic waves at an electric cylinder torque of 232-266 N and a welding time of 0.290-0.370 s, and the last chamber is sealed by welding using ultrasonic waves at an air cylinder pressure of 2.0-2.5 bar and a welding time of 0.16-0.22 s to form the container tail (7); optionally, each sealing part (2) is formed by weak welding using ultrasonic waves at an amplitude of 10%-42% and a welding time of 0.19-0.64 s, preferably at an amplitude of 36%-39% and a welding time of 0.47-0.49 s, and the last chamber is sealed by welding under welded seal conditions of an amplitude not less than 43% and a welding time not less than 0.65 s to form the container tail (7).

15. The method for manufacturing the eye drops product according to any one of claims 13-14, wherein an external force capable of resulting in irreversible detachment of the sealing part does not exceed an average pinch force of fingers of children aged 5-7 years, preferably not exceeding 17-27 Newtons (N).

16. The multi-chamber container according to any one of claims 13-15, wherein a heat seal strength of the sealing part (2) is not less than 3 N, not less than 5 N, not less than 7 N, not less than 10 N, not less than 12 N, not less than 14 N, or not less than 16 N, and is not more than 62 N, not more than 60 N, not more than 58 N, not more than 55 N, not more than 51 N, not more than 45 N, not more than 40 N, not more than 38 N, not more than 34 N, or not more than 30 N, and is preferably 3-60 N, 5-58 N, 7-58 N, 10-55 N, 12-51 N, or 16-38 N.
